# EUROPÄISCHE PATENTSCHRIFT

(11) **EP 1 599 154 B1**
(45) Veröffentlichungstag und Bekanntmachung des Hinweises auf die Patenterteilung: **06.06.2012**
(21) Anmeldenummer: 04714266.6
(22) Anmeldetag: 25.02.2004
(51) Int. Cl.: A61F 2/38

(54) **KNIEGELENKENDOPROTHESE**
KNEE-JOINT ENDOPROSTHESIS
PROTHESE D'ARTICULATION DU GENOU

(30) Priorität: 25.02.2003 DE 10307889
(43) Veröffentlichungstag der Anmeldung: 30.11.2005
(73) Patentinhaber: MTM Medizintechnik Mauk GmbH, 22844 Norderstedt (DE)
(72) Erfinder: MAUK, Rudolf, 22844 Norderstedt (DE)
(74) Vertreter: Hansmann, Dierk
(86) Internationale Anmeldenummer: PCT/DE2004/000347
(87) Internationale Veröffentlichungsnummer: WO 2004/075793

(56) Entgegenhaltungen:
- EP-A- 1 169 980
- FR-A- 2 726 174
- FR-A- 2 797 392
- US-B1- 6 203 576

## Beschreibung

Die Erfindung betrifft eine Kniegelenkendoprothese mit einem Femurteil und einem Tibiateil, die gegeneinander verschwenkbar um eine Schwenkachse gelagert sind, wobei der Tibiateil ein Tibiaplateau mit zwei Gleitpartnern aufweist, von denen der eine Gleitpartner eine Lagerplatte bildet und der andere als ein auf der Lagerplatte um eine Führung schwenkbeweglich gelagertes Gleitteil als Gleitpartner ausgebildet ist, auf dem ein Femurteil verschwenkbar lagerbar ist, und bei dem die Führung als eine fest mit dem einen Gleitpartner verbundene Erhöhung ausgebildet ist, die in eine im anderen Gleitpartner vorgesehene Vertiefung zur passenden Aufnahme hineinragt und in dieser geführt ist.

Eine Kniegelenkendoprothese besteht im Regelfall aus einem mit einer Tibia zu verbindenden Tibiateil, und einem auf diesem verschwenkbar gelagerten Femurteil, der mit einem Femur verbunden wird. Dabei ist der Femurteil beim Ausführen von Schwenkbewegungen in einer Beugeebene auf einem Gleitteil gelagert, das beispielsweise aus einem Polyethylen besteht. Dieses Gleitteil ist auf einem Tibiaplateau befestigt, das auf seiner dem Femurteil zugewandten Seite eine Lagerplatte besitzt, auf der das Gleitteil gelagert ist. Dieses Gleitteil kann fest mit der Lagerplatte verbunden sein oder gleitend auf dieser gelagert sein.

Im Falle einer gleitenden Lagerung des Gleitteils wird dieses auf dem Tibiaplateau geführt. Diese Führung kann so gestaltet sein, dass im Bereich der üblicherweise im Kniegelenk auftretenden Schwenkbewegungen ein Abheben des Gleitteils von der Lagerplatte ausgeschlossen ist. Zu diesem Zwecke erhebt sich auf der Lagerplatte eine Erhöhung, um die das Gleitteil auf der Lagerplatte verschwenkbar gelagert ist. Dabei ragt ein die Erhöhung überkragender Flansch in eine entsprechende Ausnehmung des Gleitteils hinein. In diese Verriegelungsstellung gelangt das Gleitteil mit einer entsprechend vorgesehene Vertiefung nur in einer bestimmten,Verechwenklage, die das Gleitteil gegenüber der Lagerplatte einnimmt und die außerhalb eines Bewegungsapielraumes liegt, der vom Gleitteil gegenüber der Lagerplatte beim üblichen Einsatz der Kniegelenkendoprothese eingehalten wind.

Bei einem derartigen Tibiaplateau kann das Gleitteil nur in einer bestimmten Stellung gegenüber der Lagerplatte auf diese aufgesetzt werden. Außerhalb dieser Stellung wird die auf der Lagerplatte vorgesehene Erhöhung nicht von der Vertiefung aufgenommen. Während der Operation muß der Arzt beim Einsetzen des Kniegelenks nicht selten mehrere Verechwenketellungen des Gleitteils gegenüber der Lagerplatte ausprobieren, bevor das Gleitteil auf der Lagerplatte sicher geführt wird. Die Gefahr besteht, dass beim schnellen Aufsetzen des Gleitteils während einer Operation die Erhöhung nicht sauber in die Vertiefung hineingleitet, so dass das Gleitteil sich in einer nicht zulässigen Montagestellung befindet und damit die Funktion der gesamten Knieendoprothese stört.

Nach der FR-A-2 797 392 ist eine gattungsgemäße Kniegelenkendoprothese bekannt. Hierbei ist die Einbaulage vorgegeben und der Bewegungswinkel der Gleitpartner begrenzt. Ferner ist eine fest vorgegebene Schwenkachse und Arretierung der Teile zueinander nicht gegeben.

Auch nach der FR-A-2 726 174 ist eine Anordnung dieser Art bekannt, wobei ebenfalls keine fixierte Schwenkachse gebildet wird.

Aufgabe der vorliegenden Erfindung ist es daher, eine Kniegelenkendoprothese der einleitend genannten Art so zu verbessern, dass das Gleitteil sicher und schnell auf der Lagerplatte in einer funktional richtigen Stellung gelagert werden kann.

Die Aufgabe wir erfindungsgemäß dadurch gelöst, daß eine Verriegelung der Gleitpartner über einen um die Erhöhung sich erstreckenden und mit dieser verbundenen Zahnkranz mit vorstehenden Zahnelementen gebildet ist, die in Ausnehmungen eines in der Vertiefung umgebenden Randbereiches einsetzbar sind und in einen Drehbewegungen des Zahnkranzes zulassenden in Einsetzrichtung nachgeordneten Freiraum hineingreifen sowie nach einer Verdrehung der Gleitpartner gegeneinander die zahnelemente in einem zwischen den Ausnehmungen liegenden Abdeckbereich verriegelt sind.

Ein solches Tibiaplateau besitzt eine Vielzahl von Stellungen des Gleitteils, in denen dieses so in Richtung auf die Lagerplatte abgesenkt werden kann, dass das Gleitteil im physiologischen Gleitbereich sicher auf der Laserplatte geführt wird und eine unbeabsichtigte Trennung des Gleitteils von der Lagerplatte verhindert wird. Damit wird die Montage des Tibiaplateaus erheblich vereinfacht und die Sicherheit steigt, dass das Gleitteil richtig auf der Lagerplatte aufliegt. Der die Montage des Tibiaplattaus leitende Arzt kann seine Aufmerksamkeit anderen wesentlichen Aufgaben widmen, die er während der Operation durchzuführen hat. Auch eine mehrmalige Funktionsüberprüfung des eingebauten Tibiaplateaus erweist sich als überflüssig.

Gemäß einer bevorzugten Ausführungsform der Erfindung weisen die Ausnehmungen eine ähnliche Gestaltung ihres Querschnitts wie die Elemente auf. Auch durch diese Maßnahme erfolgt eine enge Führung der beiden aufeinander zu lagernden Teile, so dass diese nur in einer für die Funktion richtigen Lage zusammengefügt werden können.

Gemäß einer weiteren bevorzugten Ausführungsform der Erfindung sind die Ausnehmungen über dem Randbereich in gleicher Dichte und gleichem Abstand verteilt wie die Elemente über dem Kranz. Durch diese Maßnahme erhöht sich die Anzahl der Stellungen, in denen eine richtige Montage der zusammenzufügenden Teile zu erwarten ist. Auch dadurch erhöht sich nicht nur die Sicherheit der Montage sondern auch die Schnelligkeit ihrer DurchFührung.

Gemäß einer weiteren bevorzugten Ausführungsform der Erfindung ragen die zahnförmigen Ausnehmungen in den Freiraum, der eine den Zahnkranz hinsichtlich seiner Dicke und seines Durchmessers aufnehmenden Querschnitt aufweist. Auf diese Weise kommt unterhalb der Lagerplatte eine sichere Verriegelung des Zahnkranzes zustande. Der Zahnkranz ist innerhalb des Freiraumes verschwenkbar gelagert.

Gemäß einer weiteren bevorzugten Ausführungsform der Erfindung ist die Erhöhung in einem dem Gleitteil benachbarten Bereich auf Zahnkonturen geführt, die die Vertiefung im Bereich der Lagerplatte begrenzen. Diese Zahnkonturen führen das Gleitteil bei Drehungen gegenüber der Lagerplatte und erlauben ein einfaches Aufsetzen und Demontieren des Gleitteils gegenüber dem Zahnkranz.

Der Femurteil einer Kniegelenkendoprothese ist häufig als ein Femurlagerteil ausgebildet, mit dem ein Stiel lösbar verbunden ist. Dabei trägt die Anordnung des Stiels auf dem Femurlagerteil der natürlichen Valgusstellung des Oberschenkels dadurch Rechnung, daß er um einen der Valgusstellung entsprechenden Winkel nach lateral geneigt ist. Diese Neigung kommt dadurch zustande, daß die Verbindung zwischen dem Femurlagerteil und dem Stiel so ausgebildet ist, daß nach der Verbindung des Stiels mit.dem Femurlagerteil der Stiel die der Valgusstellung entsprechenden Neigung aufweist.

Zu diesem Zwecke sind häufig Konusverbindungen eingesetzt worden. In diesem Falle wird auf dem Femurlagerteil ein Konus befestigt, dessen Längsachse die gewünschte Neigung aufweist. Auf diesen Konus wird ein im Stiel vorgesehener Innenkonus aufgesetzt, so daß anschließend der Stiel die der Valguastellung entsprechende Neigung aufweist.

Diese Anordnung führt dazu, daß eine Vielzahl von Femurlagerteilen vorrätig gehalten werden muß, von denen jedes die jeweils gewünschte Valgusstellung ermöglicht. Da die Femurlagerteile im Hinblick auf eine Vielzahl von Arbeitsschritten, die mit höchster Präzision ausgeführt werden müssten, sehr teuer sind, mußte eine erhebliche Lagerhaltung vorgehalten werden, um den jeweils benötigten Bedarf von Patienten decken zu können.

Dabei ist insbesondere zu berücksichtigen, daß auch noch unterschiedliche Femurlagerteile vorgehalten werden müssen entsprechend der Verwendung für ein auf der rechten Seite zu implantierenden Kniegelenkes oder für ein Kniegelenk, das auf der linken Seite implantiert werden soll.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

In den Zeichnungen zeigen :

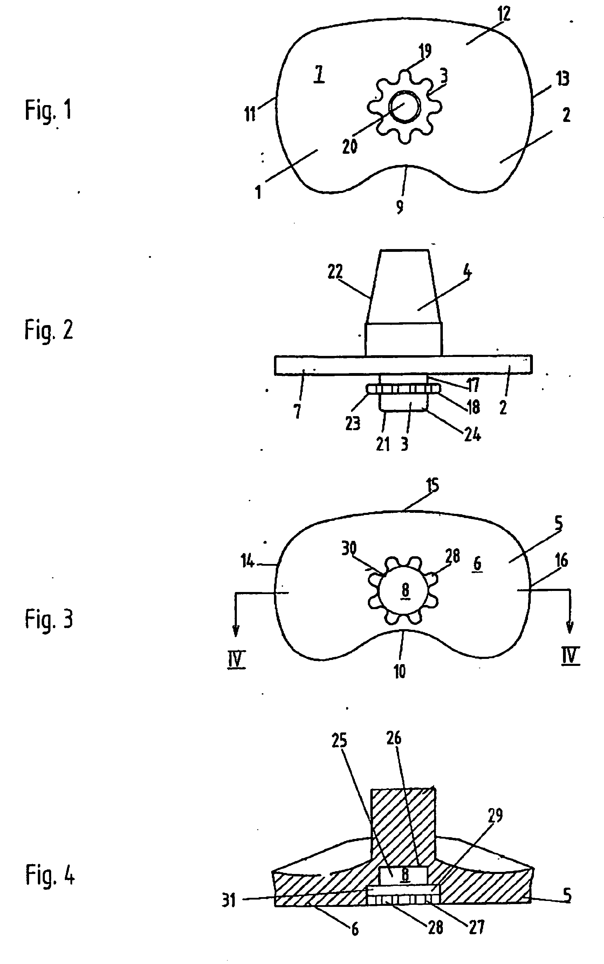

- Figur 1: eine Draufsicht auf eine Lagerplatte eines ersten Gleitpartners,
- Figur 2: eine Seitenansicht eines ersten Gleitpartners,
- Figur 3: eine Draufsicht auf eine Gleitfläche eines zweiten Gleitpartners und
- Figur 4: einen Querschnitt durch einen zweiten Gleitpartner entsprechend der Schnittlinie IV-IV in Figur 3.

Ein erster Gleitpartner (1) ist als ein Tibiateil, einer Kniegelenkendoprothese ausgebildet. Dieses Tibiateil besteht im wesentlichen aus einer Lagerplatte (2), einer Erhöhung (3) und einem Befestigungsstutzen (4) zur Befestigung des ersten Gleitpartners (1) an einer nicht dargestellten Tibia eines ebenfalls nicht dargestellten menschlichen Beins.

Darüber hinaus besteht das Tibiateil aus einem zweiten Gleitpartner (5), der mit einer Gleitfläche (6) im zusammengebauten Zustand des Tibiaplateaus die Lagerplatte (2) auf deren dem Befestigungsstutzen (4) abgewandten Druckfläche (7) beaufschlagt.

Die beiden Gleitpartner (1, 5) sind um die Erhöhung (3) verschwenkbar gegeneinander gelagert. Zu diesem Zwecke befindet sich innerhalb der Gleitfläche (6) eine Vertiefung (8), in die die Erhöhung (3) passend hineinragt. Die Erhöhung (3) und die Vertiefung (8) sind so in ihren jeweiligen Gleitpartnern (1, 5) angeordnet, dass in einer Ausgangsstellung die beiden Gleitpartner (1, 5) mit ihren jeweiligen Einwölbungen (9, 10) und ihren Begrenzungslinien (11, 12, 13, 14, 15, 16) etwa parallel zueinander verlaufen. Dabei dienen die Einwölbungen (9, 10) dazu, einem Stiel eines Femurteils die notwendige Bewegungefreiheit einzuräumen, die dieser bei Beugebewegungen des Kniegelenkes benötigt.

Die Erhöhung (3) ist in einem bestimmten Abstand (17) von der Druckfläche (7) von eine Zahnkranz (18) ringförmig umgeben, der fest mit der Oberfläche der Erhöhung verbunden ist. Auf den Zahnkranz (18) befinden sich Zähne (19), die sternförmig von der Oberfläche der Erhöhung(3) weg nach außen ragen.

Sowohl der Befestigungsstutzen (4) als auch die Erhöhung (3) sind von einer Bohrung (20) durchzogen, die einerseits in ein der Druckfläche (7) abgewandtes Ende (21) der Erhöhung (3) und andererseits in ein konisches Endteil (22) des Befestigungsstutzens (4) endet. Die Erhöhung (3) besitzt auf einer dem Ende (21) zugewandten Unterseite (23) des Zahnkranzes (18) ein Führungsstück (x24), das in eine ihm angepaßte Aufnahme (25) hineinragt, die die Vertiefung (8) an ihrem am weitesten in den zweiten Gleitpartner (5) hineinragenden Ende (26) begrenzt. In dieser Aufnahme (25) wird das Führungsstück (24) und damit der gesamte zweite Gleitpartner (5) geführt.

Die Vertiefung (8) besitzt im Bereich ihrer Einmündung (27) in die Gleifläche (6) zahnförmige Ausnehmungen (28), die die Einmündung (27) sternförmig umgeben. Diese zahnförmigen Ausnhemungen (28) sind in ihrer Formgebung den Zähnen (19) des Zahnkranzes (18) weitgehend nachgebildet, so daß beim Zusammenfügen der beiden Gleitpartner (1,5) die Zähne (19) durch die Zabnförmigen Ausnehmungen (28) hindurch gleiten können, bis die Gleitfläche (6) des zweiten Gleitpartner (5) auf der Druckfläche (7) der Lagerplatte (2) aufliegt. Dieser Stellung haben die Zähne (19) die zahnförmigen Ausenhmungen (28) pasiert und ragen in einen Freiraum (29) hinein, der den Abmessungen des Zahnkranzes (18) entsprechend groß bemessen ist, so daß beim verschwenkten der beiden Gleitpartner (1,5) gegeneinander der Zahnkranz sich innerhalb des Freiraums (29) bewegen kann. Zu diesem Zwecke ist der Freiraum (29) als ein die Vertiefung (8) umgebender Ringraum ausgebildet, dessen Querschnitt im Zahnkranz hinsichtlich seiner Dicke und seines Durchmessers angepaßt ist.

Die Vertiefung (8) wird im Bereich der Lagerplatte von einem Randbereich begrenzt, der im wesentlichen aus Zahnkonturen (30) besteht, die die Vertiefung (8) begrenzen. Auf diesen Zahnkonturen wird in die Vertiefung (8) eingeführte Erhöhung (3) im Bereich des Abstandes (17) geführt.

Zum Zwecke einer Montage wird der erste Gleitpartnern (1) im Bereich des Befestigungsstutzens (4) mit einer nicht dargestellten Tibia verbunden. Sodann wird der zweite Gleitpartner (5) mit seiner Vertiefung (8) über das Ende (21) der Erhöhung (3) geführt. Sollten die Zähne (19) nicht ummittelbar nach dem Aufsetzen des zweiten Gleitpartners (5) durch die zahnförmigen Ausnehmungen (28) hindurch gleiten, in Richtung auf den Freiraum (29), wird der zweite Gleitpartner (5) ein wenig verschwenkt, so daß die Zähne (19) in die Lage versetzt werden, durch die zahnförmigen Ausnehmungen (28) in Richtung auf den Freiraum (29) hindurch zu gleiten. Zu diesem Zwecke sind die zahnförmigen Ausnehmungen (28) weitgehend in ihrer Gestaltung den Zähnen (19) des Zahnkranzes (18) angepaßt.

Sobald der Zahnkranz (18) den Freiraum (29) erreicht hat, wird der zweite Gleitpartner (5) gegenüber dem ersten Gleitpartner (1) ein wenig verschwenkt, so daß nunmehr die Zähne (19) nicht mehr in einer Flucht mit den zahnförmigen Ausnehmungen (28) verlaufen sondern am Verlassen des Freiraums (29) durch Flanken (31) gehindert werden, die sich zwischen den einzelnen zahnförmigen Ausnehmungen (28) erstrecken und als Führung für die Zähne (19) dienen.

Um ein reibungsloses Einführen der Zähne (19) in die zahnförmigen Ausnehmungen (28) gewährleisten zu können, besitzen die zahnförmigen Ausnehmungen (28) die selbe Anordnung innerhalb der Einmündung (27) der Vertiefung (8) wie die Zähne (19) auf dem Zahnkranz (18). Diese Anordnung ist dadurch gekennzeichnet, daß zwei sich bezüglich einer Mittellinie der Erhöhung gegenüberliegende Zähne auf einer gemeinsamen Mittellinie verlaufen. Ebenso verlaufen auch zwei gegenüber liegende angeordnete zahnförmige Ausnehmungen (28) auf einer gemeinsamen Mittellinie. Schließlich sind über den Umfang sowohl der Erhöhung (3) als auch der Vertiefung (8) eine gleiche Anzahl von Zähnen (19) beziehungsweise zahnförmigen Ausnehmungen (28) angeordnet. Allerdings sind bezüglich einander parallel verlaufender Begrenzungslinien (11, 12, 13 ; 14, 15, 16) die zahnförmigen Ausnehmungen (28) um einen Winkel von 22,5° verschoben, so daß die Zähne (19) bei parallel verlaufenden Begrenzungslinien (11, 12, 13 ; 14, 15, 16) nicht durch die zahnförmigen Ausnehmungen (28) hindurch bewegt werden können, sondern von den Flanken (31) im Freiraum (29) gehalten werden. Dabei kann der Zahnkranz (18) innerhalb des Freiraums (29) um einen Gesamtwinkel von 45° verschwenkt werden, ohne daß die Gefahr besteht, daß die Zähne (19) des Zahnkranzes (18) durch die zahnförmigen Ausnehmungen (28) hindurch gleiten können. Andererseits sind jedoch über den gesamten Umfang des Zahnkranzes (15) acht Möglichkeiten vorgesehen, den Zahnkranz (15) zwischen den zahnförmigen Ausnehmungen (28) hindurch in den Freiraum (29) einführen zu können. Dadurch wird deutlich, daß das Aufsetzen des zweiten Gleitpartners (5) auf die Lagerplatte (2) des ersten Gleitpartners (1) schnell und ohne Zeitaufwand für das Ausprobieren der richtigen Ineinanderfügung der Gleitpartner (1,5) durchgeführt werden kann. Außerdem ist dafür gesorgt, daß der zweite Gleitpartner (5) ohne Schwierigkeiten auf die Lagerplatte (2) aufgesetzt werden kann, ohne daß dabei das Risiko besteht, daß die beiden Gleitpartner (1,5) sich nicht in der vorgesehenen Lage einander beaufschlagen. Die Montage der beiden Gleitpartner erfolgt daher schnell und ohne das Risiko, daß die beiden Gleitpartner (1,5) sich nicht in der gewünschten Weise beaufschlagen. Vielmehr liegt die Gleitfläche (6) auf der Lagerplatte (2) großflächig auf.

## Patentansprüche

1. Kniegelenkendoprothese mit einem Femurteil und einem Tibiateil, die gegeneinander verschwenkbar um eine Schwenkachse gelagert sind, wobei der Tibiateil ein Tibiaplateau mit zwei Gleitpartnern (1, 5) aufweist, von denen der eine Gleitpartner (1) eine Lagerplatte (2) bildet und der andere als ein auf der Lagerplatte (2) um eine Führung schwenkbeweglich gelagertes Gleitteil als Gleitpartner (5) ausgebildet ist, auf dem ein Femurteil verschwenkbar lagerbar ist, und bei dem die Führung als eine fest mit dem einen Gleitpartner (1) verbundene Erhöhung (3) ausgebildet ist, die in eine im anderen Gleitpartner (5) vorgesehene Vertiefung (8) zur passenden Aufnahme hineinragt und in dieser geführt ist, **dadurch gekennzeichnet, daß** eine Verriegelung der Gleitpartner (1, 5) über einen um die Erhöhung (3) sich erstreckenden und mit dieser verbundenen Zahnkranz (18) mit vorstehenden Zahnelementen (19) gebildet ist, die durch Ausnehmungen (28) eines die Vertiefung (8) umgebenden Randbereiches hindurch einsetzbar sind und in einen Drehbewegungen des Zahnkranzes (18) zulassenden, in Einsetzrichtung nachgeordneten Freiraum (29) hineingreifen, und daß nach einer Verdrehung der Gleitpartner (1, 5) gegeneinander die Zahnelemente (19) in einem zwischen den Ausnehmungen (28) liegenden Abdeckbereich verriegelt sind.

2. Kniegelenkendoprothese nach Anspruch 1, **dadurch gekennzeichnet, daß** die Ausnehmungen (28) eine ähnliche Gestaltung ihrer Querschnitte wie die Zahnelemente (19) aufweisen.

3. Kniegelenkendoprothese nach Anspruch 1 oder 2, **dadurch gekennzeichnet, daß** die Ausnehmungen (28) über den Randbereich in gleicher Dichte und in gleichem Abstand verteilt sind wie die Zahnelemente (19) über den Kranz (18).

4. Kniegelenkendoprothese nach einem der Anspruch 1 bis 3, **dadurch gekennzeichnet, daß** die zahnförmigen Ausnehmungen (28) in den Freiraum (29) hineinragen, der eine den Zahnkranz (18) hinsichtlich seiner Dicke und seines Durchmessers aufnehmenden Querschnitt aufweist.

5. Kniegelenkendoprothese nach einem der Ansprüche 1 bis 4, **dadurch gekennzeichnet, daß** der Freiraum (29) als ein sich um die Vertiefung (8) erstreckender Ringraum ausgebildet ist.

6. Kniegelenkendoprothese nach einem der Ansprüche 1 bis 5, **dadurch gekennzeichnet, daß** der Randbereich aus zahnkonturen (30) besteht, die die Vertiefung (8) im Bereich der Lagerplatte (2) begrenzen.

7. Kniegelenkendoprothese nach Anspruch 6, **dadurch gekennzeichnet, daß** die Erhöhung (3) in einem dem Gleitpartner (1) benachbarten Bereich auf den Zahnkonturen (30) geführt ist.

8. Kniegelenkendoprothese nach Anspruch 7, **dadurch gekennzeichnet, daß** zwischen dem Zahnkranz (18) und dem ihr benachbarten Gleitpartner (1) ein Abstandsbereich (17) vorgesehen ist, dessen Höhe einer Wandstärke des zahnbereiche entspricht, der in dem Abstandsbereich (17) mit enger Passung weitgehend spielfrei gelagert ist.

9. Kniegelenkendoprothese nach einem der Ansprüche 1 bis 8, **dadurch gekennzeichnet, daß** die Erhöhung (3) auf einer dem Abstandsbereich (17) abgelegenen Seite des Zahnkränzes (18) einen Endbereich (21) aufweist, der in einem über den Freiraum (29) hinausragenden tiefsten Bereich der Vertiefung (8) geführt ist.

10. Kniegelenkendoprothese nach einem der Anspruche 1 bis 9 **dadurch gekennzeichnet, daß** die zähne (19) bezüglich der zahnförmigen Ausnehmungen (28) so angeordnet sind, daß in einer Grundeinstellung der einander beaufschlagenden Gleitpartner (1,5) sich der Gleitpartner (5) nicht von der Lagerplatte (2) abziehen läßt.

11. Kniegelenkendoprothese nach Anspruch 10, **dadurch gekennzeichnet, daß** in der Grundstellung der Gleitpartner {1,5} eine zur Aufnahme eines verschwenkten Femure im Gleitpartner (5) vorgesehene Einwölbung (10) parallel zu einer entsprechenden Einwölbung (9) - der Lagerplatte (2) verläuft.

12. Kniegelenkendoprothese nach einem der Ansprüche 1 bis 11, **dadurch gekennzeichnet, daß** jeweils zwei einander im Zahnkranz (18) sich gegenüberliegende Zähne (19) gleich ausgerichtete Mittellinien aufweisen.

13. Kniegelenkendoprothese nach Anspruch 12, **dadurch gekennzeichnet, daß** die Mittellinien zweier einander gegenüberliegenden Zähne (19) mit den Mittellinien zweier einander gegenüberliegender zahnförmigen Ausnehmungen (2B) bei parallel verlaufenden Einwölbungen (9,10) von Gleitteil (5) einerseits und Lagerplatte (2) andererseits einen Winkel von etwa 20° bis 40° einschließen.

14. Kniegelenkendoprothese nach Anspruch 13, **dadurch gekennzeichnet, daß** der Winkel zwischen den Mittellinien von einander gegenüber verlaufenden Zähnen (19) und den Mittellinien von einander gegenüberliegend verlaufenden zahnförmigen Ausnehmungen (28) bei gleicher Ausrichtung der Gleitpartner (1) 22,5° beträgt.

## Claims

1. Knee-joint endoprosthesis having a femur part and a tibia part which are mounted so as to be pivotable against one another about an axis of swivelling, wherein the tibia part has a tibia plateau with two sliding partners (1, 5), of which one sliding partner (1) forms a bearing plate (2) and the other is constructed as a sliding part, in the form of a sliding partner (5), which is mounted on the bearing plate (2) for swivelling movement about a guide and on which a femur part can be mounted in a pivotable manner, and in which the guide is constructed as an elevation (3) which is fixedly connected to the one sliding partner (1) and which protrudes into a depression (8) provided in the other sliding partner (5) for receiving it so as to fit, and is guided in said depression, **characterised in that** a locking arrangement of the sliding partners (1, 5) is formed via a toothed ring (18) which extends around the elevation (3) and is connected to the latter and which has projecting tooth elements (19) which can be inserted through clearances (28) in an edge region surrounding the depression (8) and engage in a free space (29) which permits rotational movements of the toothed ring (18) and is arranged downstream in the direction of insertion, and that, after twisting of the sliding partners (1, 5) against one another, the tooth elements (19) are locked in an enveloping region lying between the clearances (28).

2. Knee-joint endoprosthesis according to claim 1, **characterised in that** the clearances (28) have a configuration of their cross-sections which is similar to the tooth elements (19).

3. Knee-joint endoprosthesis according to claim 1 or 2, **characterised in that** the clearances (28) are distributed over the edge region in the same density and at the same distance apart as the tooth elements (19) are distributed over the ring (18).

4. Knee-joint endoprosthesis according to one of claims 1 to 3, **characterised in that** the tooth-shaped clearances (28) protrude into the free space (29) which has a cross-section which receives the toothed ring (18) with respect to its thickness and its diameter.

5. Knee-joint endoprosthesis according to one of claims 1 to 4, **characterised in that** the free space (29) is constructed as an annular space which extends around the depression (8).

6. Knee-joint endoprosthesis according to one of claims 1 to 5, **characterised in that** the edge region consists of toothed contours (30) which delimit the depression (8) in the region of the bearing plate (2).

7. Knee-joint endoprosthesis according to claim 6, **characterised in that** the elevation (3) is guided on the toothed contours (30) in a region adjacent to the sliding partner (1).

8. Knee-joint endoprosthesis according to claim 7, **characterised in that** there is provided, between the toothed ring (18) and the sliding partner (1) adjacent to said prosthesis, a spacing region (17), the height of which corresponds to a wall thickness of the toothed region which is mounted in the spacing region (17) with a close fit and in a largely play-free manner.

9. Knee-joint endoprosthesis according to one of claims 1 to 8, **characterised in that** the elevation (3) has, on a side of the toothed ring (18) remote from the spacing region (17), an end region (21) which is guided in a deepest region of the depression (8) that protrudes above the free space (29).

10. Knee-joint endoprosthesis according to one of claims 1 to 9, **characterised in that** the teeth (19) are so arranged, as regards the tooth-shaped clearances (28), that the sliding partner (5) cannot be removed from the bearing plate (2) when the sliding partners (1, 5) that act upon one another are in a basic setting.

11. Knee-joint endoprosthesis according to claim 10, **characterised in that**, when the sliding partners (1, 5) are in the basic position, a concavity (10), which is provided in the sliding partner (5) for receiving a pivoted femur, extends parallel to a corresponding concavity (9) in the bearing plate (2).

12. Knee-joint endoprosthesis according to one of claims 1 to 11, **characterised in that** any two teeth (19) which are mutually opposed in the toothed ring (18) have identically oriented median lines.

13. Knee-joint endoprosthesis according to claim 12, **characterised in that** the median lines of two mutually opposed teeth (19) form an angle of about 20° to 40° with the median lines of two mutually opposed tooth-shaped clearances (28) when concavities (9, 10) in the sliding part (5), on the one hand, and in the bearing plate (2), on the other hand, extend parallel.

14. Knee-joint endoprosthesis according to claim 13, **characterised in that** the angle between the median lines of teeth (19) that extend in a mutually opposite manner and the median lines of tooth-shaped clearances (28) that extend in a mutually opposed manner is 22.5° when the sliding partners (1) are oriented in an identical manner.

## Revendications

1. Endoprothèse d'articulation du genou, avec un composant fémoral et un composant tibial, qui sont montés à pivotement, l'un par rapport à l'autre, autour d'un axe, sachant que le composant tibial est doté d'un plateau de tibia avec deux partenaires de glissement (1, 5), l'un (1) des partenaires de glissement formant une plaque de support (2) et l'autre partenaire de glissement (5) étant réalisé sous la forme d'une pièce de glissement montée, sur la plaque de support (2), à pivotement autour d'un guidage, et sur laquelle un composant fémoral peut être monté à pivotement, sachant que le guidage est réalisé sous la forme d'une élévation (3), qui s'engage, en ajustage précis, dans une cavité (8), prévue dans l'autre partenaire de glissement (5), et est guidée dans celle-ci, **caractérisée en ce qu'**un verrouillage des partenaire de glissement (1, 5) est réalisé par l'intermédiaire d'une couronne dentée (18), qui s'étend autour de l'élévation (3), à laquelle elle est reliée, et qui est dotée d'éléments dentés (19) saillants, qui peuvent passer à travers des évidements (28) d'une zone marginale entourant la cavité (8), et qui s'engagent dans un espace libre (29) situé en aval, dans la direction d'introduction, permettant un mouvement de rotation de la couronne dentée (18), et **en ce que**, après un pivotement relatif des partenaires de glissement (1, 5), les éléments dentés (19) sont verrouillés dans une zone de recouvrement, située entre les évidements (28).

2. Endoprothèse d'articulation du genou selon la revendication 1, **caractérisée en ce que** les évidements (28) présentent des sections transversales de conception similaire à celles des éléments dentés (19).

3. Endoprothèse d'articulation du genou selon revendication 1 ou 2, **caractérisée en ce que** les évidements (28) sont répartis, sur la zone marginale, avec la même densité et le même espacement que les éléments dentés (19) sur la couronne dentée (18).

4. Endoprothèse d'articulation du genou selon l'une des revendications 1 à 3, **caractérisée en ce que** les évidements (28), en forme de dents, font saillie dans l'espace libre (29), qui est doté d'une section transversale permettant de recevoir l'épaisseur et le diamètre de la couronne dentée (18).

5. Endoprothèse d'articulation du genou selon l'une des revendications 1 à 4, **caractérisée en ce que** l'espace libre (29) est conçu sous la forme d'un espace annulaire, qui s'étend autour de la cavité (8).

6. Endoprothèse d'articulation du genou selon l'une des revendications 1 à 5, **caractérisée en ce que** la zone marginale est dotée de contours dentés (30), qui limitent la cavité (8) dans la région de la plaque de support (2).

7. Endoprothèse d'articulation du genou selon la revendication 6, **caractérisée en ce que** l'élévation (3) est guidée sur les contours dentés (30), dans une zone voisine du partenaire de glissement (1).

8. Endoprothèse d'articulation du genou selon la revendication 7, **caractérisée en ce que**, entre la couronne dentée (18) et le partenaire de glissement (1) voisin d'elle, est prévue une zone d'écartement (17), dont la hauteur correspond à une épaisseur de paroi de la zone dentée, qui est montée, étroitement ajustée, exempte de jeu dans la plus grande mesure possible, dans la zone d'écartement (17).

9. Endoprothèse d'articulation du genou selon l'une des revendications 1 à 8, **caractérisée en ce que** l'élévation (3) est dotée, sur un côté de la couronne dentée (18) éloigné de la zone d'écartement (17), d'une zone d'extrémité (21), qui est amenée dans une la zone la plus profonde de la cavité (8), au-delà de l'espace libre (29).

10. Endoprothèse d'articulation du genou selon l'une des revendications 1 à 9, **caractérisée en ce que** les dents (19), sont disposées, par rapport aux évidements en forme de dents (28) de sorte que, dans une position de base des partenaires de glissement (1,5) emboîtés, le partenaire de glissement (5) ne puisse pas être arraché de la plaque de support (2).

11. Endoprothèse d'articulation du genou selon la revendication 10, **caractérisée en ce que**, dans la position de base des partenaires de glissement (1, 5) une concavité (10), prévue, dans le partenaire de glissement (5) pour recevoir un fémur pivoté, s'étend parallèlement à une concavité (9) correspondante de la plaque de support (2).

12. Endoprothèse d'articulation du genou selon l'une des revendications 1 à 11, **caractérisée en ce que**, chaque fois, deux dents (19), situées l'une en face de l'autre dans la couronne dentée (18), présentent des lignes médianes de même orientation.

13. Endoprothèse d'articulation du genou selon la revendication 12, **caractérisée en ce que** les lignes médianes de deux dents (19) opposées forment avec les lignes médianes de deux évidements en forme de dents (28) opposés un angle de 20 ° à 40 ° environ, les concavités (9, 10) du partenaire de glissement (5), d'une part, et de la plaque de support (2), d'autre part, s'étendant parallèlement.

14. Endoprothèse d'articulation du genou selon la revendication 13, **caractérisée en ce que** l'angle entre les lignes médianes de dents (19) situées l'une en face de l'autre et les lignes médianes d'évidements en forme de dents (28), situés à l'opposé l'un de l'autre, est de 22,5 °, les partenaires de glissement (1, 5) étant orientés de manière identique.
